# EUROPEAN PATENT SPECIFICATION

(11) **EP 3 909 513 B1**
(45) Date of publication and mention of the grant of the patent: **11.09.2024**
(21) Application number: 19908105.0
(22) Date of filing: 26.12.2019
(51) Int. Cl.: A61B 17/34, A61B 8/12, A61M 25/06, A61M 25/09, A61B 90/00, A61B 17/00, A61B 10/04

(54) **ENDOSCOPE PUNCTURE DEVICE**
ENDOSKOPISCHE PUNKTIONSVORRICHTUNG
DISPOSITIF DE PERFORATION D'ENDOSCOPE

(30) Priority: 09.01.2019 JP 2019001713
(43) Date of publication of application: 17.11.2021
(73) Proprietor: Zeon Corporation, Tokyo 100-8246 (JP)
(72) Inventor: GON, Chimyon, Tokyo 100-8246 (JP)
(74) Representative: Maiwald GmbH
(86) International application number: PCT/JP2019/051170
(87) International publication number: WO 2020/145181

(56) References cited:
- WO-A1-2017/210656
- WO-A1-2020/016981
- JP-A- 2000 262 629
- JP-A- 2000 262 629
- JP-A- 2015 002 920
- US-A1- 2010 048 990
- US-A1- 2015 265 302
- US-A1- 2016 157 839
- US-A1- 2016 157 886

## Description

### TECHNICAL FIELD

The present invention relates to an endoscope puncture device.

### BACKGROUND ART

In recent years, there have been some reports in which endoscopic ultrasound-guided biliary drainage (EUS-BD) is performed in a case where, for example, the transduodenal papilla approach is impossible with biliary drainage required due to unresectable malignant biliary stenosis or obstruction. In the EUS-BD procedure, an ultrasonic endoscope is inserted into the digestive tract and the common bile duct (or intrahepatic bile duct) is punctured with a puncture needle from the inside of the digestive tract such as the duodenum, stomach, and esophagus during real-time ultrasonic image observation. After a guide wire is inserted into the bile duct via the puncture hole, a tubular object that serves as a bypass path connecting the inside of the digestive tract and the inside of the common bile duct (or intrahepatic bile duct) is inserted and placed along the guide wire. This procedure enables biliary drainage in the form of tubular object embedding in the body.

In some cases, a self-expanding stent provided with a covering film is used as the tubular object that is used as a bypass path in EUS-BD. A stent delivery device is used in this case, and a known example of the device includes a catheter having an inner sheath and an outer sheath through which the inner sheath is slidably inserted. The stent is disposed in a stent disposition portion provided in the vicinity of the distal end of the inner sheath. The stent is held, with its diameter reduced, inside the vicinity of the distal end of the outer sheath. On the proximal end side of the catheter, the outer sheath is slid with respect to the inner sheath so as to be pulled out. As a result, the diameter of the stent is expanded.

In addition, an endoscope puncture needle (hereinafter, puncture needle) may be used during, for example, intrahepatic bile duct puncturing from the stomach wall. The puncture needle, an example of which is described in Patent Document 1, is for the purpose of body tissue collection from a target site in a patient's body. The puncture needle described in Patent Document 1 is a hollow puncture needle provided with a blade surface at its tip. The collected body tissue can be suctioned through the lumen of the puncture needle. In the EUS-BD procedure, the lumen of the puncture needle is used as an insertion hole for guide wire insertion. The following procedure is performed by means of an endoscope puncture device with the puncture needle disposed in a sheath inserted into the treatment tool guide channel of an endoscope.

For example, in the case of bypass connection between the stomach and the intrahepatic bile duct, the sheath is inserted into the treatment tool guide channel of the endoscope, the tip portion of the puncture needle is pushed out by a predetermined length from the tip of the sheath protruding from the tip of the endoscope, and stomach and liver puncturing is performed so as to reach the intrahepatic bile duct from the inside of the stomach. Next, a path is secured by a guide wire being inserted through the lumen of the puncture needle, pushed out, and inserted so as to serve as a bridge between the puncture hole of the stomach and the puncture hole of the liver. Next, with the tip portion of the puncture needle pulled in the sheath, the sheath is pulled out of the treatment tool guide channel of the endoscope along the guide wire so that damage to the inside of the endoscope and so on are avoided. Subsequently, the stent delivery device is prepared, a catheter tube is pushed out along the guide wire, and the site where the stent of the catheter tube is disposed is disposed so as to serve as a bridge between the puncture holes. By the stent being released (expanded) in this state, the stent is placed so as to serve as a bridge between the stomach and the bile duct.

However, in a case where the EUS-BD procedure is performed by means of the endoscope puncture device, various operations such as pushing, pulling, and rotating the guide wire with the guide wire inserted in the lumen of the puncture needle may lead to contact between the guide wire and the blade surface of the puncture needle and damage to the guide wire since the blade surface is formed at the tip of the puncture needle.

US 2010/048990 A1 discloses an endoscopic needle comprising a distal protecting stylet having a channel configured to retain the guide wire.

### CITATION LIST

### PATENT DOCUMENT

Patent Document 1: JP 2016-513998 A
Patent Document 2: US 2010/048990 A1

### SUMMARY OF THE INVENTION

### PROBLEM TO BE SOLVED BY THE INVENTION

The present invention has been made in view of such circumstances, and an object of the present invention is to provide an endoscope puncture device having a puncture needle capable of preventing damage to a guide wire.

### MEANS FOR SOLVING PROBLEM

In order to achieve the above object, the endoscopic puncture device according to the present invention has:
a sheath suitable to be inserted into an endoscope;
a hollow puncture needle disposed in the sheath and provided with a blade surface at a tip thereof; and
a hollow resin molded body provided in the puncture needle on a distal side of the puncture needle and at least partially disposed inside a tip opening portion of the puncture needle, wherein
the resin molded body has a through hole through the resin molded body to insert a guide wire and an opening portion formed at the distal end of the through hole, and
characterized in that
an edge portion of the opening portion is disposed along an edge portion of the tip opening portion and continuously extends along an entire circumference of the edge portion of the tip opening portion.

The endoscope puncture device according to the present invention has the hollow resin molded body provided in the puncture needle and at least partially disposed inside the tip opening portion of the puncture needle. Accordingly, even when various operations such as pushing, pulling, and rotating a guide wire are performed with the guide wire inserted in the lumen of the puncture needle, the guide wire comes into contact with the resin molded body and does not come into contact with the blade surface of the puncture needle (edge part of the blade surface in particular) inside the tip opening portion of the puncture needle. Accordingly, the guide wire can be protected from the blade surface of the puncture needle and damage to the guide wire can be prevented.

A guide wire is insertable through the resin molded body and the resin molded body preferably has a predetermined wall thickness such that the guide wire inserted therethrough is centered with respect to a central axis of the puncture needle. In the case of such a configuration, the guide wire that is inserted through the resin molded body is disposed offset toward the radial center portion of the puncture needle by the wall thickness of the resin molded body. Accordingly, inside the tip opening portion of the puncture needle, the guide wire can be disposed in a substantially central portion of the puncture needle in the radial direction (at a position far from the blade surface of the puncture needle) and the guide wire can be effectively protected from the blade surface of the puncture needle.

Preferably, the resin molded body has an inclined portion inclined in accordance with inclination of the blade surface. When the inclined portion is disposed inside the tip opening portion of the puncture needle in such a configuration, the inclined portion is disposed along the edge portion of the tip opening portion inside the tip opening portion of the puncture needle. Accordingly, most of the inside of the tip opening portion of the puncture needle can be covered with the resin molded body and the guide wire can be effectively protected from the blade surface of the puncture needle.

Preferably, an inner diameter of the resin molded body decreases toward a tip side of the resin molded body. In this case, the guide wire inserted through the resin molded body is disposed in a substantially central portion of the puncture needle in the radial direction toward the tip side of the resin molded body. Accordingly, inside the tip opening portion of the puncture needle, the guide wire can be easily centered with respect to the central axis of the puncture needle.

A tapered surface or a step portion may be formed on an outer peripheral surface of the resin molded body. With such a configuration, it is possible to prevent the resin molded body from falling out of the puncture needle.

### BRIEF DESCRIPTION OF DRAWINGS

Fig. 1A is a front view illustrating the overall configuration of a stent delivery device;
Fig. 1B is a front view illustrating the overall configuration of an endoscope puncture device according to a first embodiment of the present invention;
Fig. 2A is a partially enlarged perspective view of a puncture needle included in the endoscope puncture device illustrated in Fig. 1B;
Fig. 2B is a perspective view illustrating a resin molded body provided in the puncture needle illustrated in Fig. 2A;
Fig. 3A is a vertical cross-sectional view of the resin molded body illustrated in Fig. 2B;
Fig. 3B is a vertical cross-sectional view of the resin molded body provided in the puncture needle included in the endoscope puncture device according to a second embodiment of the present invention;
Fig. 3C is a vertical cross-sectional view of the resin molded body provided in the puncture needle included in the endoscope puncture device according to a third embodiment of the present invention;
Fig. 4A is a vertical cross-sectional view of the resin molded body provided in the puncture needle included in the endoscope puncture device according to a fourth embodiment of the present invention;
Fig. 4B is a vertical cross-sectional view of the resin molded body provided in the puncture needle included in the endoscope puncture device according to a fifth embodiment of the present invention;
Fig. 4C is a vertical cross-sectional view of the resin molded body provided in the puncture needle included in the endoscope puncture device according to a sixth embodiment of the present invention;
Fig. 5 is an enlarged vertical cross-sectional view at a time when a guide wire is inserted through the puncture needle illustrated in Fig. 2A;
Fig. 6 is a diagram illustrating work (puncturing) at a time of placing a stent in a puncture hole by means of the stent delivery device illustrated in Fig. 1A;
Fig. 7 is a diagram illustrating work (guide wire insertion) at the time of placing the stent in the puncture hole by means of the stent delivery device illustrated in Fig. 1A;
Fig. 8 is a diagram illustrating work (catheter insertion initiation) at the time of placing the stent in the puncture hole by means of the stent delivery device illustrated in Fig. 1A;
Fig. 9 is a diagram illustrating work (catheter insertion completion) at the time of placing the stent in the puncture hole by means of the stent delivery device illustrated in Fig. 1A;
Fig. 10 is a diagram illustrating work (during stent release) at the time of placing the stent in the puncture hole by means of the stent delivery device illustrated in Fig. 1A; and
Fig. 11 is a diagram illustrating work (stent release completion) at the time of placing the stent in the puncture hole by means of the stent delivery device illustrated in Fig. 1A.

### MODE(S) FOR CARRYING OUT THE INVENTION

### First Embodiment

Hereinafter, embodiments of the present invention will be specifically described with reference to the drawings. Described as an example in the present embodiment is a case where the stomach and liver are punctured from the inside of the stomach to the intrahepatic bile duct and a self-expanding stent provided with a covering film for bypass connection between the stomach and the intrahepatic bile duct is placed in the puncture hole in a case where endoscopic ultrasound-guided biliary drainage (EUS-BD) is performed in a transgastric and transhepatic manner. However, the present invention is not limited to the bypass connection between the stomach and the intrahepatic bile duct and can be widely applied during bypass connection between a luminal organ and another luminal organ such as the duodenum and the common bile duct.

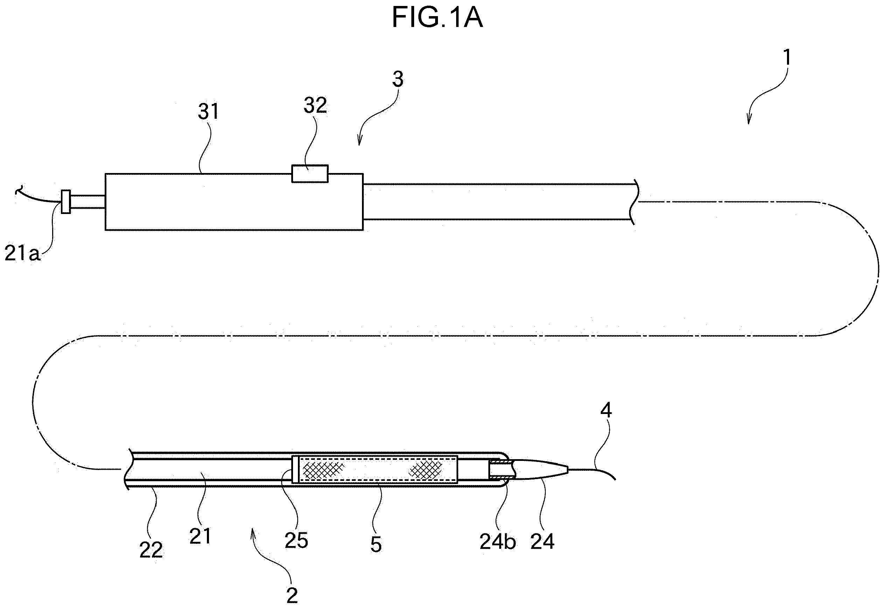

As illustrated in Fig. 1A, a stent delivery device 1 is configured to substantially include an elongated catheter portion 2 inserted into a patient's body (lumen) via the treatment tool guide channel of an endoscope (not illustrated), an operation unit 3 connected to the proximal end side of the catheter portion 2 so that the catheter portion 2 in the body is operated from outside the body, a guide wire 4, and a stent 5 as an object of placement. It should be noted that the vicinity of the distal end of the catheter portion 2 including the stent 5 is drawn linearly for convenience in the drawing although it may be curved depending on the shape of the site where the placement occurs.

The catheter portion 2 includes an inner sheath (inner tube) 21 having a distal end and a proximal end and an outer sheath (outer tube) 22 having a distal end and a proximal end. A contrast marker (not illustrated) is attached to the vicinity of the distal end of each of the inner sheath 21 and the outer sheath 22. The contrast marker serves as a marker in the body with its position detected by X-ray fluoroscopy. The contrast marker is formed of, for example, a metal material such as gold, platinum, and tungsten or a polymer blended with barium sulfate or bismuth oxide.

The inner sheath 21 is made of an elongated and flexible tube. The guide wire 4 used as a guide for inserting the catheter portion 2 into the patient's body is inserted through the lumen of the inner sheath 21. After inserting the guide wire 4 into the body and securing a path between the outside and the inside of the body, it is possible to insert the distal end side of the catheter portion 2 into a target site in the body by pushing in (advancing) the catheter portion 2 along the guide wire 4. The outer diameter of the inner sheath 21 (part where the stent 5 is disposed (described later)) is approximately 0.5 to 3.5 mm.

A leading end tip 24 is attached to the distal end of the inner sheath 21. The leading end tip 24 is formed so as to decrease in thickness toward the tip (distal end) thereof. The leading end tip 24 has an opening communicating with the distal end of the lumen of the inner sheath 21 along the central axis thereof. In addition, the proximal end portion of the leading end tip 24 is a small-diameter portion 24b having an outer diameter at which insertion into the distal end portion of the lumen of the outer sheath 22 is possible. The leading end tip 24 reduces irritation to the internal lumen portion, reduces the insertion resistance of the catheter portion 2, and facilitates insertion into the body in a case where the distal end of the inner sheath 21 (catheter portion 2) abuts against the peripheral wall of the internal lumen portion. The leading end tip 24 can be formed of, for example, polyamide, polyamide elastomer, polyurethane, fluororesin such as polytetrafluoroethylene, polyolefin resin such as polyethylene, or the like.

A fixing ring 25 is integrally fixed in the vicinity of the distal end of the inner sheath 21. The fixing ring 25 is to define the position of the proximal end of the stent 5. The part on the distal end side that is from the fixing ring 25 is the stent disposition portion. The stent 5 is disposed in the stent disposition portion so as to cover the inner sheath 21. In addition, another elongated tube (not illustrated) is coaxially provided at the part of the inner sheath 21 that is closer to the proximal end side than the fixing ring 25 so as to cover the elongated tube constituting the inner sheath 21 main body. The part of the inner sheath 21 closer to the proximal end side than the fixing ring 25 is thicker than the part of the inner sheath 21 that is closer to the distal end side than the fixing ring 25. Since the part of the inner sheath 21 closer to the proximal end side than the fixing ring 25 is thick as described above, the pushability of the inner sheath 21 increases to improve operability and a positional deviation of the fixing ring 25 to the proximal end side is prevented.

The outer sheath 22 is made of an elongated and flexible tube and has an inner diameter slightly larger than the outer diameter of the inner sheath 21. The inner sheath 21 is slidably inserted through the inside of the outer sheath 22. The inner diameter of the outer sheath 22 is approximately 0.8 to 3.5 mm, and the outer diameter of the outer sheath 22 is approximately 1.2 to 4.0 mm. The outer sheath 22 is capable of sliding (relatively moving) in the axial direction with respect to the inner sheath 21 by the operation unit 3 being operated.

The inner sheath 21 and the outer sheath 22 can be formed of various resin materials including polyolefin such as polyethylene, polypropylene and so on; polyvinyl chloride; polyurethane; ethylene-vinyl acetate copolymer; polyester such as polyethylene terephthalate, polybutylene terephthalate and so on; polyamide; polyether polyamide; polyester polyamide; polyetheretherketone; polyetherimide; and fluorine-based resin such as polytetrafluoroethylene and tetrafluoroethylene-hexafluoropropylene copolymer; or various thermoplastic elastomers such as polystyrene-based, polyolefin-based, polyurethane-based, polyester-based, polyamide-based, and polybutadiene-based elastomers. In addition, two or more of these can be used in combination.

It should be noted that an outermost tube (not illustrated) may be coaxially disposed on the outside of the outer sheath 22 although the present embodiment lacks the outermost tube. The outermost tube is made of an elongated and flexible tube and has a lumen into which the outer sheath 22 is slidably inserted. A tube having a dimension larger than the outer diameter of the outer sheath 22 by approximately 0.05 to 1.0 mm can be used as the outermost tube. Polyacetal, polytetrafluoroethylene, tetrafluoroethylene-hexafluoropropylene copolymer, polypropylene, and so on can be used as the material of the outermost tube.

The stent 5 is a self-expanding covered stent that expands from a contracted state by its own elastic force. The stent 5 has a tubular bare stent formed by a frame and a covering film portion covering the outer periphery of the bare stent. The bare stent is formed of, for example, shape memory metal or superelastic metal such as nickel-titanium alloy, cobalt-chromium alloy, gold-titanium alloy, and beta-titanium alloy. The surface of the bare stent is covered with a covering film that spreads so as to fill the space between adjacent frames. The outer periphery of the bare stent covered with the covering film is covered with the covering film portion such as a polymer film.

Although the total length of the stent 5 is determined in accordance with the distance between luminal organs to be bypass-connected, the total length is approximately 30 to 200 mm. Although the outer diameter of the stent 5 at the time of diameter expansion is determined in accordance with, for example, the type and size of the luminal organ to be bypass-connected, the outer diameter is approximately φ2 to φ30 mm. The outer diameter of the stent 5 at the time of diameter reduction is approximately a fraction with respect to the external shape at the time of diameter expansion. It should be noted that the stent 5 can be appropriately replaced depending on the purpose although the stent 5 in the present embodiment is described as a member constituting the stent delivery device 1.

As illustrated in Fig. 1A, the guide wire 4 is used as a guide for inserting the catheter portion 2 into the patient's body, is inserted through the lumen of the inner sheath 21, and has a distal end protruding from the opening at the distal end of the leading end tip 24 of the inner sheath 21. The proximal end of the guide wire 4 is disposed so as to be exposed to the outside via an opening 21a at the proximal end of the inner sheath 21 disposed so as to penetrate the operation unit 3. Although a wire having a diameter of 0.035 inches (approximately 0.889 mm), a wire having a diameter of 0.025 inches (approximately 0.635 mm), or the like is generally used as the guide wire, a wire having a diameter of 0.025 inches is used as the guide wire 4 in the present embodiment.

The operation unit 3 has a substantially cylindrical release handle (housing) 31. The opening portion on the distal end side of the release handle 31 is blocked by a distal end side lid member being integrally attached. The distal end side lid member has a through hole in the middle portion thereof. The opening portion on the proximal end side of the release handle 31 is blocked by a distal end side lid member being integrally attached. The distal end side lid member has a through hole in the middle portion thereof.

The proximal end portion of the outer sheath 22 is slidably inserted through the through hole of the distal end side lid member of the release handle 31. The proximal end of the outer sheath 22 is positioned in the release handle 31. In the side wall of the release handle 31, a groove is formed so as to penetrate the side wall inward and outward in the direction along the central axis thereof. A release lever 32 has a head portion positioned on the outside of the release handle 31 and a foot portion erected in the middle portion of the head portion. The foot portion of the release lever 32 is disposed in a penetrating manner in the groove.

The tip portion (lower end portion) of the foot portion of the release lever 32 is fixed to the proximal end portion of the outer sheath 22 positioned in the release handle 31. By moving the release lever 32 along the groove, it is possible to slide the outer sheath 22 to the proximal end side or the distal end side with respect to the inner sheath 21 fixed to the release handle 31 (proximal end side lid member).

The proximal end portion of the inner sheath 21 inserted through the outer sheath 22 passes through the release handle 31 and penetrates the through hole of the proximal end side lid member of the release handle 31, and the proximal end thereof is positioned outside the release handle 31. The inner sheath 21 is fixed to the proximal end side lid member (release handle 31) at the part of the through hole.

In a state where the release lever 32 is moved to the distal end of the groove, the distal end of the outer sheath 22 reaches the leading end tip 24 and the small-diameter portion 24b of the leading end tip 24 is inserted in the distal end portion of the lumen of the outer sheath 22. In this state, the stent 5 disposed in the stent disposition portion of the inner sheath 21 is held therein in a diameter-reduced state. By the release lever 32 being moved from this state to the proximal end side of the groove, the outer sheath 22 is slid to the proximal end side with respect to the inner sheath 21, the stent 5 is relatively pushed out from the distal end of the outer sheath 22, and the stent 5 is released (expanded in diameter) by a self-expanding force.

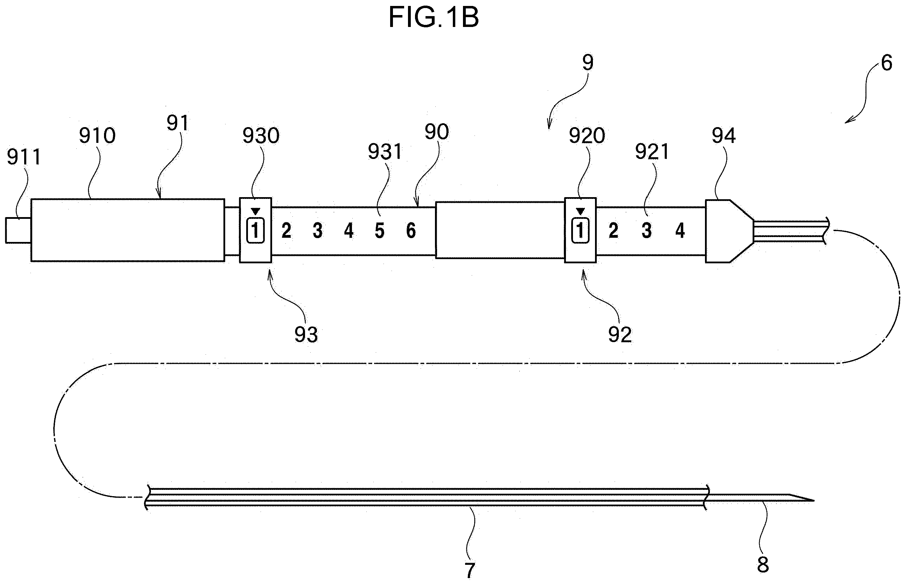

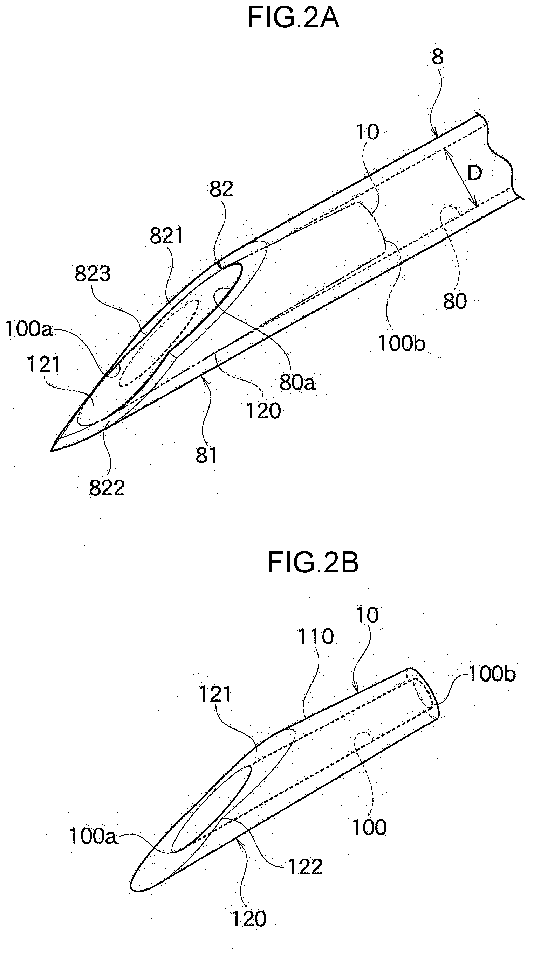

As illustrated in Figs. 1B and 2A, an endoscope puncture device 6 has a sheath 7, a puncture needle 8, an operation unit 9, and a resin molded body 10. The endoscope puncture device 6 is used when a puncture hole is formed in the peripheral wall of a luminal organ to be bypass-connected and inter-luminal organ guide wire insertion is performed via the puncture hole in the EUS-BD procedure. It should be noted that the guide wire 4 used for inserting the stent delivery device 1 can be used as it is as the guide wire used in this case.

The sheath 7 is made of an elongated and flexible tube. The puncture needle 8 is inserted through the lumen of the sheath 7. Preferably, the outer diameter of the sheath 7 is equal to or larger than the outer diameter of the puncture needle 8 (described later). The sheath 7 is inserted into the treatment tool guide channel of an endoscope (such as an ultrasonic endoscope, not illustrated) and is capable of sliding (relatively moving) in the axial direction with respect to the treatment tool guide channel of the endoscope by the operation unit 9 being operated. The materials exemplified as the materials of the inner sheath 21 and the outer sheath 22 of the stent delivery device 1 described above can be used as the material of the sheath 7.

The puncture needle 8 is made of an elongated, flexible, and cylindrical body and is inserted through (disposed in) the sheath 7 so as to be capable of moving forward and backward. As illustrated in Fig. 2A, a lumen 80 extending along the axial direction is formed in the puncture needle 8. The guide wire 4 (see Fig. 1A) can be inserted through the lumen 80.

The inner diameter of the puncture needle 8 (a diameter D of the lumen 80) is, for example, 0.3 to 1.3 mm. The outer diameter of the puncture needle 8 is, for example, 0.4 to 1.7 mm. Preferably, a flexible metal material such as stainless steel and a nickel-titanium alloy or the like is used as the material of the puncture needle 8. In the present embodiment, stainless steel is used as the material that constitutes the entire puncture needle 8.

The puncture needle 8 has a puncture portion 81. The puncture portion 81 has a sharp shape and is formed in the tip portion (distal end portion) of the puncture needle 8. A blade surface 82 inclined with respect to the axis of the puncture needle 8 is formed in the puncture portion 81. When the puncture portion 81 is pressed against a body wall, it is possible to provide a puncture hole in the body wall.

The blade surface 82 has a refracting portion 823, a first blade surface 821 positioned on the base end side (proximal side) of the refracting portion 823, and a second blade surface 822 positioned on the tip side (distal side) of the refracting portion 823. The blade surfaces 821 and 822 are discontinuously connected via the refracting portion 823. The first blade surface 821 and the second blade surface 822 have different inclination angles with respect to the axis of the puncture needle 8. A part of the second blade surface 822 is curved while being inclined at an inclination angle larger than that of the first blade surface 821.

A tip opening portion 80a is formed at the tip of the puncture needle 8. The blade surface 82 is formed along the edge portion of the tip opening portion 80a. In the present embodiment, the tip of the puncture needle 8 is inclined with respect to the axis thereof, and thus the tip opening portion 80a has a substantially elliptical shape.

Although not illustrated in detail, a plurality of annular grooves may be provided in the surface of the tip portion of the puncture needle 8 at a predetermined position. With such a configuration, the ultrasonic image of the tip portion of the puncture needle 8 is clearly depicted on an observation image and it is possible to accurately measure the positional relationship and distance between the puncture needle 8 and the puncture site that is punctured by the puncture needle 8.

As illustrated in Fig. 1B, the operation unit 9 is made of, for example, a resin material and has an operation unit main body 90 where the base end portion of the sheath 7 is fixedly provided and a slider 91 provided so as to be slidable with respect to the operation unit main body 90.

The slider 91 is a cylindrical body and has a slider main body 910 gripped by a user and a small-diameter portion 911 coaxially formed in the base end portion (proximal end portion) thereof. The base end portion of the puncture needle 8 is fixedly provided in the small-diameter portion 911.

The operation unit main body 90 is made of an elongated and cylindrical body and has a sheath moving portion 92, a needle moving portion 93, and a connecting portion 94. The connecting portion 94 is fixed to the tip portion of the operation unit main body 90 by means such as adhesion, and the base end portion (distal end portion) of the sheath 7 is fixedly provided. The connecting portion 94 is connected and fixed to the inlet of the treatment tool guide channel of an endoscope (not illustrated). As a result, the endoscope puncture device 6 can be fixed to the endoscope.

The sheath moving portion 92 is a mechanism for adjusting the positional relationship between the endoscope (not illustrated) and the sheath 7 and is provided on the tip side (distal end side) of the operation unit main body 90. The sheath moving portion 92 has a stopper 920 and a scale portion 921. The stopper 920 is disposed so as to be slidable with respect to the operation unit main body 90. The scale portion 921 has a plurality of scales that serve as an index when the disposition position of the stopper 920 is set. The scales are disposed at predetermined intervals on the outer peripheral surface of the operation unit main body 90. Each scale indicates the length of protrusion of the sheath 7 from the tip of the endoscope (not illustrated). For example, when the stopper 920 is disposed at the scale position that corresponds to the numerical value of " 1" and the slider 91 is slid to the distal side in that state with the connecting portion 94 connected and fixed to the endoscope, it is possible to cause the tip of the sheath 7 to protrude from the tip of the endoscope by the length indicated by the scale (that is, by 1 cm).

The needle moving portion 93 is a mechanism for adjusting the positional relationship between the sheath 7 and the puncture needle 8 and is provided on the base end side (proximal end side) of the operation unit main body 90. The needle moving portion 93 has a stopper 930 and a scale portion 931. The stopper 930 is disposed so as to be slidable with respect to the operation unit main body 90. The scale portion 931 has a plurality of scales that serve as an index when the disposition position of the stopper 930 is set. The scales are disposed at predetermined intervals on the outer peripheral surface of the operation unit main body 90. Each scale indicates the length of protrusion of the puncture needle 8 from the tip of the sheath 7. For example, when the stopper 930 is disposed at the scale position that corresponds to the numerical value of "1" and the slider 91 is slid to the distal side in that state, it is possible to cause the tip of the puncture needle 8 to protrude from the tip of the sheath 7 by the length indicated by the scale (that is, by 1 cm).

As illustrated in Fig. 2A, the resin molded body 10 has a hollow shape and is fitted in the puncture needle 8 (lumen 80). The resin molded body 10 has an outer diameter substantially equal to the diameter of the lumen 80 of the puncture needle 8. The outer peripheral surface of the resin molded body 10 is fixed to the inner peripheral surface of the puncture needle 8. The outer peripheral surface of the resin molded body 10 is fixed to the inner peripheral surface of the puncture needle 8 by adhesion, press-fitting, or the like. Adhesion by means of an adhesive is used in the present embodiment.

The resin molded body 10 is made of a material softer than the material constituting the puncture needle 8. The resin molded body 10 is made of a resin material such as polyethylene, polyamide, polyurethane, polypropylene, polyacetal, polyetheretherketone, polytetrafluoroethylene, and tetrafluoroethylene-hexafluoropropylene copolymer. In addition, injection molding is preferable as the molding method for molding the resin molded body 10 from the resin material although known resin material molding methods can be used without particular limitation as the molding method.

As illustrated in Fig. 2B, the resin molded body 10 has a base end portion 110 and a tip portion 120. The base end portion 110 is disposed on the base end side of the resin molded body 10 and is a substantially cylindrical part. The tip portion 120 is disposed on the tip side of the resin molded body 10 and is a part having a sharp shape.

The tip portion 120 has a shape obtained by a linear resin member molded in a substantially cylindrical shape being cut at an angle with respect to the axis. The tip portion 120 has an inclined portion (inclined surface) 121 in the tip portion thereof, and the inclined portion 121 is inclined in accordance with the inclination of the blade surface 82 of the puncture portion 81. The shape of the tip portion 120 corresponds (is substantially identical) to the shape of the puncture portion 81. In the inclined portion 121, a curved portion 122 is formed at a position corresponding to the refracting portion 823 of the blade surface 82. The curved portion 122 may be omitted.

A through hole 100, which is a cylindrical space penetrating the resin molded body 10 in the axial direction, is formed in the resin molded body 10. The guide wire 4 (see Fig. 1A) can be inserted through the through hole 100.

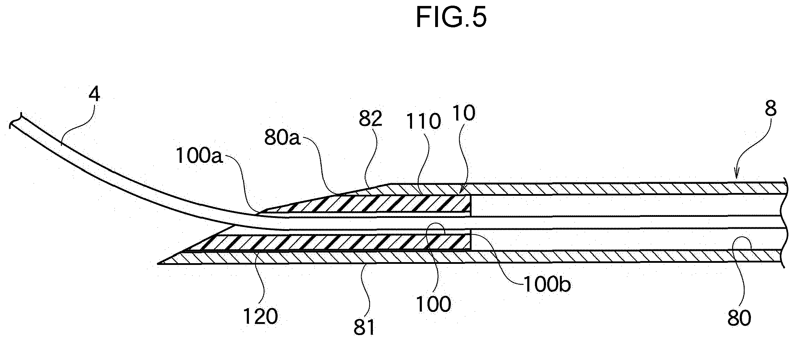

An opening portion 100a is formed at the tip of the resin molded body 10, and an opening portion 100b is formed at the base end of the resin molded body 10. The through hole 100 is connected to the lumen 80 of the puncture needle 8 via the opening portion 100b. As illustrated in Fig. 5, the guide wire 4 that is inserted through the lumen 80 can be inserted through the through hole 100 and pulled out of the resin molded body 10 from the opening portion 100a.

The diameter of the through hole 100 is smaller than the diameter D of the lumen 80 illustrated in Fig. 2A and is equal to or larger than the diameter of the guide wire 4 that is inserted through the through hole 100. Preferably, the through hole 100 has a diameter of 0.2 to 1.0 mm.

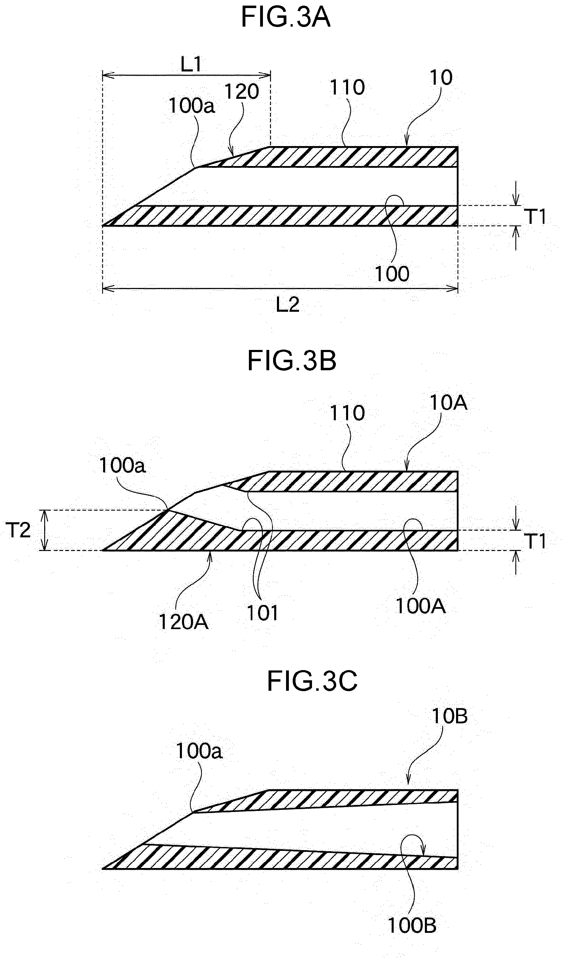

As illustrated in Fig. 3A, the resin molded body 10 has a predetermined wall thickness such that the guide wire 4 (not illustrated) that is inserted through the resin molded body 10 is centered with respect to the central axis of the puncture needle 8 illustrated in Fig. 2A. The ratio T1/D of a thickness T1 of the resin molded body 10 illustrated in Fig. 3A to the diameter D of the lumen 80 of the puncture needle 8 illustrated in Fig. 2A is preferably 1/8 to 1/3. By the value of the ratio T1/D being set within the range, the guide wire 4 that is inserted through the resin molded body 10 (through hole 100) is disposed in a substantially central portion of the puncture needle 8 in the radial direction (at a position separated by the thickness T1 from the inner peripheral surface of the puncture needle 8 to the center side in the radial direction) and is centered with respect to the central axis of the puncture needle 8. It should be noted that the thickness T1 is constant along the axial direction of the resin molded body 10.

A length L1 of the tip portion 120 along the axial direction is determined in accordance with the length of the puncture portion 81 along the axial direction. The length L1 is usually 1 to 10 mm. Preferably, a length L2 of the resin molded body 10 along the axial direction is 10 to 150 mm. The ratio L1/L2 of the length L1 to the length L2 is preferably 1/75 to 1/2 and more preferably 1/50 to 1/5.

As illustrated in Fig. 2A, the tip portion 120 is disposed in the puncture portion 81. More specifically, the tip portion 120 is disposed in the puncture portion 81 such that the position of the opening portion 100a and the position of the tip opening portion 80a are substantially the same (aligned). Accordingly, the position of the blade surface 82 and the position of the inclined portion 121 are substantially the same (aligned) and the blade surface 82 (tip of the puncture needle 8) and the inclined portion 121 (tip of the resin molded body 10) are substantially flush with each other. In addition, inside the tip opening portion 80a, the edge portion of the opening portion 100a of the tip portion 120 is disposed along the edge portion of the tip opening portion 80a (blade surface 82).

As described above, in the present embodiment, at least a part of the resin molded body 10 (the edge portion of the opening portion 100a of the tip portion 120) is disposed inside the tip opening portion 80a of the puncture portion 81 and the outer peripheral surface of the edge portion of the opening portion 100a of the tip portion 120 abuts against the inner peripheral surface of the edge portion of the tip opening portion 80a. Accordingly, inside the tip opening portion 80a, the inner peripheral surface of the edge portion of the tip opening portion 80a is directly or indirectly covered by the edge portion of the opening portion 100a of the tip portion 120 over almost the entire circumference and the edge part of the blade surface 82 is smoothly connected to the inclined portion 121 as illustrated in Fig. 5.

It should be noted that the entire outer peripheral surface of the tip portion 120 abuts against the entire inner peripheral surface of the puncture portion 81 and almost the entire inner peripheral surface of the puncture portion 81 is directly or indirectly covered by the tip portion 120 in the present embodiment. In addition, the entire outer peripheral surface of the base end portion 110 abuts against the inner peripheral surface of a part of the puncture needle 8 positioned closer to the proximal side than the puncture portion 81 and the inner peripheral surface of the part is directly or indirectly covered by the base end portion 110.

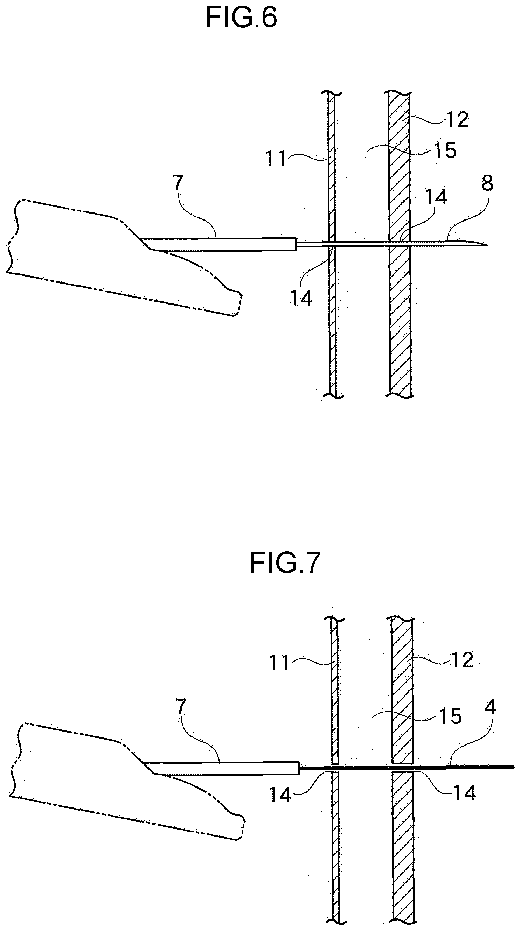

Next, a procedure for placing a stent by means of the endoscope puncture device and the stent delivery device will be described with reference to Figs. 6 to 11. First, the endoscope puncture device 6 illustrated in Fig. 1B is prepared, the sheath 7 is inserted into the treatment tool guide channel of an endoscope (not illustrated) with the puncture needle 8 disposed in the sheath 7, the connecting portion 94 of the operation unit 6 is connected and fixed to the inlet of the treatment tool guide channel of the endoscope, and the endoscope puncture device 6 is fixed to the endoscope. It should be noted that a stylet is inserted in advance into the lumen 80 of the puncture needle 8.

Next, the stopper 920 of the sheath moving portion 92 is disposed at a desired scale position of the scale portion 921, the slider 91 is slid to the distal side, and the tip of the sheath 7 is caused to protrude from the tip of the endoscope by the distance that corresponds to the numerical value of the scale. For example, in a case where the stent is placed in the abdominal cavity so as to bypass-connect the stomach and the intrahepatic bile duct, the tip of the sheath 7 can be disposed at a position separated by a predetermined distance from a stomach wall 11 by the operation described above.

Next, the stopper 930 of the needle moving portion 93 is disposed at a desired scale position of the scale portion 931, the slider 91 is slid to the distal side, and the tip of the puncture needle 8 is caused to protrude from the tip of the sheath 7 by the distance that corresponds to the numerical value of the scale. In this manner, puncturing by means of the puncture needle 8 is performed so as to reach a bile duct wall (including the liver parenchyma of the liver) 12 from the stomach wall 11 through an abdominal cavity 15 as illustrated in Fig. 6 and puncture holes 14 are formed in both the stomach and the intrahepatic bile duct. Subsequently, the stylet inserted in the lumen 80 is pulled out of the puncture needle 8 and the guide wire 4 is inserted through the lumen 80. Then, the stopper 930 of the needle moving portion 93 illustrated in Fig. 1B is disposed at a desired scale position of the scale portion 931, the slider 91 is slid to the proximal side, and the tip of the puncture needle 8 is pulled in from the tip of the sheath 7 by the distance that corresponds to the numerical value of the scale. As a result, the puncture needle 8 is removed from the puncture hole 14 and a path is secured with the guide wire 4 inserted through the puncture hole 14 as illustrated in Fig. 7. It should be noted that the endoscope is indicated by a two-dot chain line in Figs. 6 to 11.

Next, the sheath 7 is pulled out of the treatment tool guide channel of the endoscope and the endoscope puncture device 6 is removed from the endoscope. It is possible to avoid damage to the inside of the endoscope and so on by pulling the sheath 7 out of the treatment tool guide channel of the endoscope with the tip of the puncture needle 8 pulled in from the tip of the sheath 7.

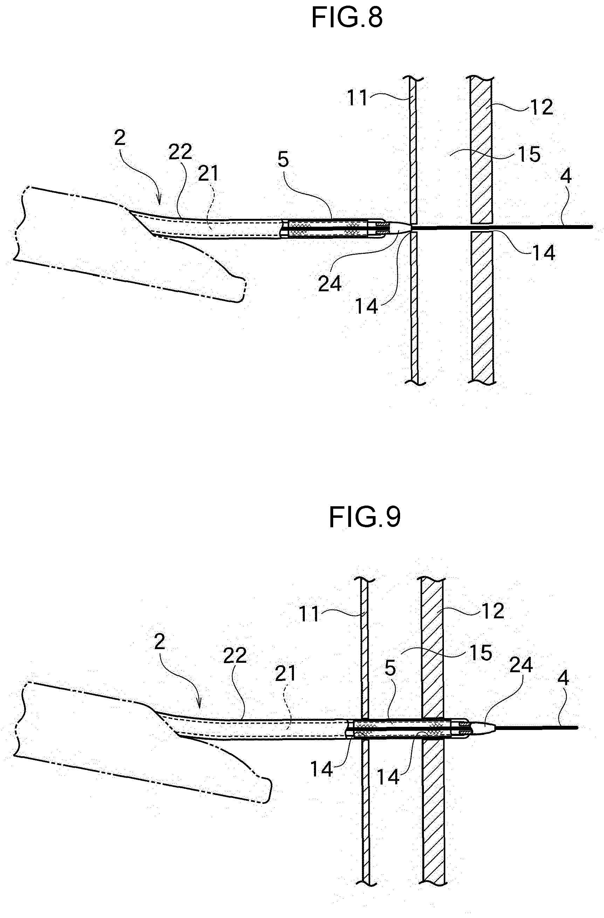

Next, the stent delivery device 1 illustrated in Fig. 1A is prepared and the puncture hole 14 is expanded by means of a dilator (not illustrated). Then, the release lever 32 of the operation unit 3 is moved to the distal end of the groove. Then, the distal end portion of the catheter portion 2 (leading end tip 24) is inserted into the puncture hole 14 on the stomach wall 11 side with the outer sheath 22 slid to the distal end side, that is, in a state where the stent 5 disposed in the stent disposition portion of the inner sheath 21 is reduced in diameter and held inside the distal end portion of the outer sheath 22 as illustrated in Fig. 8.

When the catheter portion 2 (inner sheath 21 and outer sheath 22) is inserted next such that the leading end tip 24 passes through the puncture hole 14 on the stomach wall 11 side and is inserted deeper into the abdominal cavity 15, the puncture hole 14 on the stomach wall 11 side is gradually expanded by the leading end tip 24 and the puncture hole 14 on the stomach wall 11 side is expanded by the leading end tip 24 to the extent that the distal end portion of the catheter portion 2 can be inserted. Subsequently, the catheter portion 2 is further moved forward and the distal end portion of the catheter portion 2 (leading end tip 24) is inserted into the puncture hole 14 on the bile duct wall 12 side.

When the catheter portion 2 is further moved forward, the puncture hole 14 on the bile duct wall 12 side is gradually expanded by the leading end tip 24 and the puncture hole 14 on the bile duct wall 12 side is expanded by the leading end tip 24 to the extent that the distal end portion of the catheter portion 2 can be inserted. The insertion of the catheter portion 2 is stopped with the catheter portion 2 further moved forward, the distal end of the stent 5 positioned inside the bile duct wall 12, and the proximal end of the stent 5 positioned inside the stomach wall 11 as illustrated in Fig. 9.

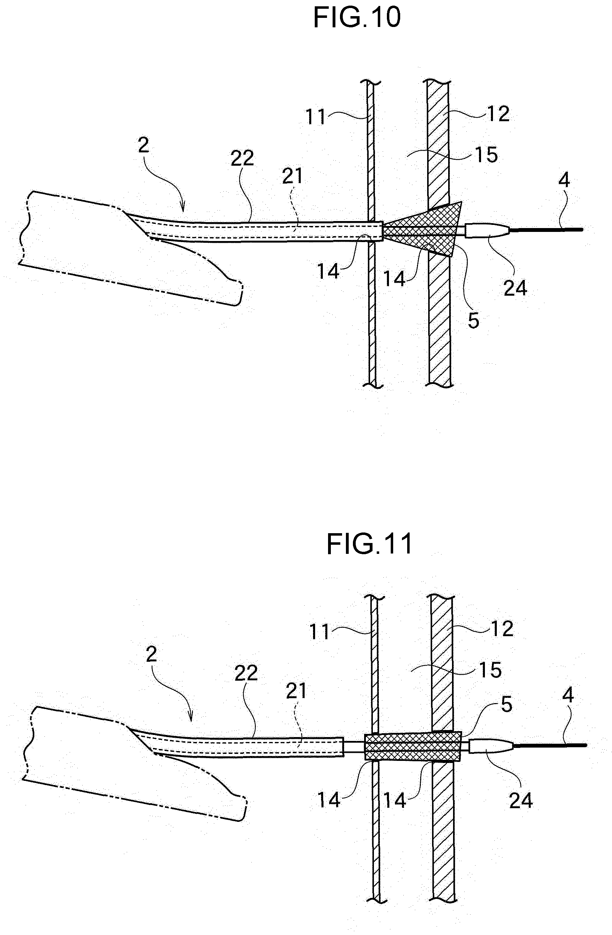

Subsequently, the release lever 32 is moved to the proximal end side of the groove in the operation unit 3 and the outer sheath 22 is slid to the proximal end side with respect to the inner sheath 21 as illustrated in Fig. 10. As the outer sheath 22 is slid to the proximal end side, the stent 5 is gradually exposed and expanded in diameter from the distal end side thereof. By the release lever 32 being moved to the proximal end of the groove, the stent 5 is exposed and expanded in diameter as a whole as illustrated in Fig. 11. Subsequently, the placement of the stent 5 is completed by the catheter portion 2 and the leading end tip 24 being passed through the diameter-expanded stent 5 and pulled out.

The endoscope puncture device 6 according to the present embodiment has the resin molded body 10. Accordingly, even when various operations such as pushing, pulling, and rotating the guide wire 4 are performed with the guide wire 4 inserted in the lumen 80 of the puncture needle 8, the guide wire 4 comes into contact with the resin molded body 10 (tip portion 120) and does not come into contact with the blade surface 82 of the puncture needle 8 (edge part of the blade surface 82 in particular) inside the tip opening portion 80a of the puncture needle 8 as illustrated in Fig. 5. Accordingly, the guide wire 4 can be protected from the blade surface 82 of the puncture needle 8 and damage to the guide wire 4 can be prevented.

In addition, the resin molded body 10 has a predetermined wall thickness such that the guide wire 4 inserted therethrough is centered with respect to the central axis of the puncture needle 8. Accordingly, the guide wire 4 that is inserted through the resin molded body 10 (through hole 100) is disposed offset toward the radial center portion of the puncture needle 8 by the wall thickness of the resin molded body 10. Accordingly, inside the tip opening portion 80a of the puncture needle 8, the guide wire 4 can be disposed in a substantially central portion of the puncture needle 8 in the radial direction (at a position far from the blade surface 82 of the puncture needle 8) and the guide wire 4 can be effectively protected from the blade surface 82 of the puncture needle 8.

In addition, the resin molded body 10 has the inclined portion 121 inclined in accordance with the inclination of the blade surface 82. Accordingly, when the inclined portion 121 is disposed inside the tip opening portion 80a of the puncture needle 8, the inclined portion 121 is disposed along the edge portion of the tip opening portion 80a inside the tip opening portion 80a of the puncture needle 8. Accordingly, most of the inside of the tip opening portion 80a of the puncture needle 8 can be covered with the resin molded body 10 and the guide wire 4 can be effectively protected from the blade surface 82 of the puncture needle 8.

### Second Embodiment

A resin molded body 10A included in the endoscope puncture device of the present embodiment illustrated in Fig. 3B is similar in configuration, action, and effect to the resin molded body 10 of the first embodiment described above except for the followings. Common parts will not be described below, and common members will be indicated by common member codes in the drawing.

As illustrated in Fig. 3B, the resin molded body 10A has a through hole 100A and a tip portion 120A. The through hole 100A is bent in a bent portion 101 positioned in the tip portion 120A and extends obliquely upward with respect to the axis of the resin molded body 10A. Although the bent portion 101 is formed in the tip portion 120A in the present embodiment, the bent portion 101 may be formed in the base end portion 110. In addition, the through hole 100A may be smoothly curved in the bent portion 101.

The wall thickness of the tip portion 120A increases toward the radial center portion toward the tip side thereof. A region having a maximum thickness T2 (thick region) is formed closer to the tip side than the bent portion 101. The maximum thickness T2 of the tip portion 120A is larger than the thickness T1 of the base end portion 110. A guide wire (not illustrated) inserted through the through hole 100A is pulled out of the opening portion 100a in a state of being offset toward the radial center portion by the distance that corresponds to the difference between the thicknesses (T2-T1).

As a result, according to the present embodiment, the guide wire can be easily centered with respect to the central axis of the puncture needle 8 and the guide wire can be effectively protected from the blade surface 82 of the puncture needle 8 inside the tip opening portion 80a of the puncture needle 8 when the resin molded body 10A is provided in the puncture needle 8 (see Fig. 2A).

### Third Embodiment

A resin molded body 10B included in the endoscope puncture device of the present embodiment illustrated in Fig. 3C is similar in configuration, action, and effect to the resin molded body 10 of the first embodiment described above except for the followings. Common parts will not be described below, and common members will be indicated by common member codes in the drawing.

As illustrated in Fig. 3C, the resin molded body 10B has a through hole 100B. The diameter of the through hole 100B (inner diameter of the resin molded body 10B) is formed so as to decrease toward the tip side of the resin molded body 10B. In addition, the resin molded body 10B is formed such that the wall thickness thereof increases toward the radial center portion toward the tip side thereof and in accordance with the shape of the through hole 100B.

Accordingly, when the resin molded body 10B is provided in the puncture needle 8 (see Fig. 2A), a guide wire (not illustrated) inserted through the resin molded body 10B is disposed in a substantially central portion of the puncture needle 8 in the radial direction toward the tip side of the resin molded body 10B. Accordingly, inside the tip opening portion 80a of the puncture needle 8, the guide wire can be easily centered with respect to the central axis of the puncture needle 8 and the guide wire can be effectively protected from the blade surface 82 of the puncture needle 8.

### Fourth Embodiment

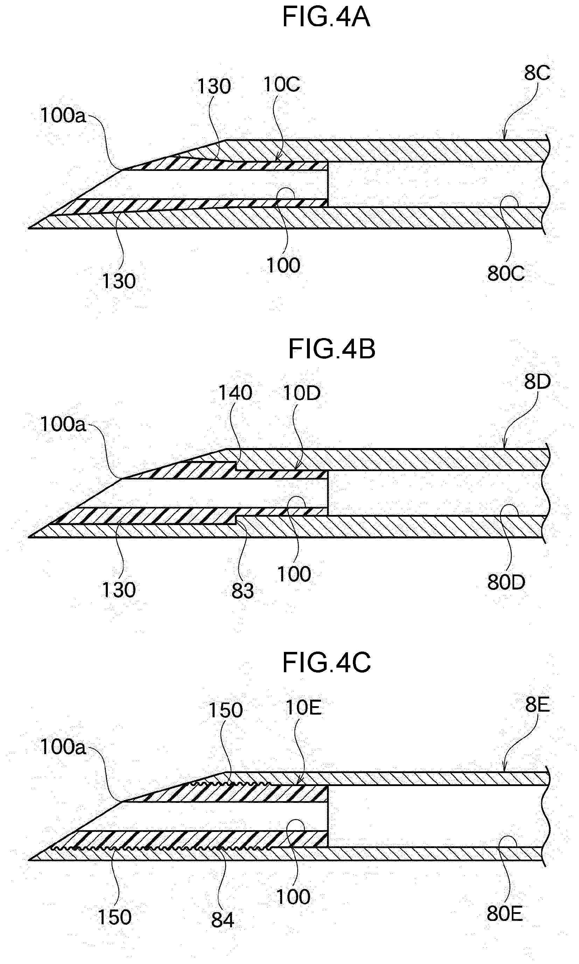

A resin molded body 10C and a puncture needle 8C included in the endoscope puncture device of the present embodiment illustrated in Fig. 4A are similar in configuration, action, and effect to the resin molded body 10 and the puncture needle 8 of the first embodiment described above except for the followings. Common parts will not be described below, and common members will be indicated by common member codes in the drawing.

As illustrated in Fig. 4A, a tapered surface 130 is formed on the outer peripheral surface of the resin molded body 10C and the tapered surface 130 is inclined so as to have an outer diameter increasing toward the tip side thereof. In addition, a lumen 80C of the puncture needle 8C is formed so as to have a diameter increasing toward the tip side of the puncture needle 8C in accordance with the shape of the outer peripheral surface of the resin molded body 10C.

In the present embodiment, the tapered surface 130 is formed on the outer peripheral surface of the resin molded body 10C. Accordingly, it is possible to prevent the resin molded body 10C from falling out of the puncture needle 8C (lumen 80C).

### Fifth Embodiment

A resin molded body 10D and a puncture needle 8D included in the endoscope puncture device of the present embodiment illustrated in Fig. 4B are similar in configuration, action, and effect to the resin molded body 10 and the puncture needle 8 of the first embodiment described above except for the followings. Common parts will not be described below, and common members will be indicated by common member codes in the drawing.

As illustrated in Fig. 4B, a step portion 140 having a substantially right-angle shape is formed on the outer peripheral surface of the resin molded body 10D. In addition, a lumen 80D of the puncture needle 8D (the inner wall surface of the puncture needle 8D) is provided with a step portion 83 that engages with the step portion 140.

In the present embodiment, the step portion 140 is formed on the outer peripheral surface of the resin molded body 10D. Accordingly, it is possible to prevent the resin molded body 10D from falling out of the puncture needle 8D (lumen 80D).

### Sixth Embodiment

A resin molded body 10E and a puncture needle 8E included in the endoscope puncture device of the present embodiment illustrated in Fig. 4C are similar in configuration, action, and effect to the resin molded body 10 and the puncture needle 8 of the first embodiment described above except for the followings. Common parts will not be described below, and common members will be indicated by common member codes in the drawing.

As illustrated in Fig. 4C, an unevenness 150 extending along the circumferential direction of the outer peripheral surface of the resin molded body 10E is formed at a part of the outer peripheral surface. In addition, a lumen 80E of the puncture needle 8E (the inner wall surface of the puncture needle 8E) is provided with an unevenness 84 that engages with the unevenness 150.

In the present embodiment, the unevenness 150 is formed on the outer peripheral surface of the resin molded body 10E. Accordingly, it is possible to prevent the resin molded body 10E from falling out of the puncture needle 8E.

It should be noted that the present invention is not limited to the embodiments described above and can be variously modified within the scope of the present invention defined in the claims.

In each of the embodiments, the shape of the resin molded body 10 is not limited to the shape illustrated in Fig. 2B or the like. The shape may be appropriately changed on condition that at least a part thereof is disposed inside the tip opening portion 8 of the puncture needle 8. For example, in Fig. 2B, the tip portion 120 alone may constitute the resin molded body 10 with the base end portion 110 omitted. Alternatively, the resin molded body 10 may have a shape (be substantially ring-shaped) that covers only the inner peripheral surface of the edge portion of the tip opening portion 80a of the puncture needle 8 (inside of the tip opening portion 80a of the puncture needle 8).

In each of the embodiments, the edge part of the blade surface 82 of the puncture needle 8 is smoothly connected to the inclined portion 121 of the resin molded body 10 as illustrated in Fig. 2A and so on. Alternatively, the part may be discontinuously connected.

### EXPLANATIONS OF LETTERS OR NUMERALS

1 STENT DELIVERY DEVICE
2 CATHETER PORTION
   21 INNER SHEATH
   22 OUTER SHEATH
   24 LEADING END TIP
      24b SMALL-DIAMETER PORTION
   25 FIXING RING
3 OPERATION UNIT
   31 RELEASE HANDLE
   32 RELEASE LEVER
4 GUIDE WIRE
5 STENT
6 ENDOSCOPE PUNCTURE DEVICE
7 SHEATH
8 PUNCTURE NEEDLE
   80 LUMEN
      80a TIP OPENING PORTION
   81 PUNCTURE PORTION
   82 BLADE SURFACE
      821 FIRST BLADE SURFACE
      822 SECOND BLADE SURFACE
      823 REFRACTING PORTION
   83 STEP PORTION
   84 UNEVENNESS
9 OPERATION UNIT
   90 OPERATION UNIT MAIN BODY
   91 SLIDER
      910 SLIDER MAIN BODY
      911 SMALL-DIAMETER PORTION
   92 SHEATH MOVING PORTION
      920 STOPPER
      921 SCALE PORTION
   93 NEEDLE MOVING PORTION
      930 STOPPER
      931 SCALE PORTION
   94 CONNECTING PORTION
10, 10A, 10B, 10C RESIN MOLDED BODY
   100, 100A, 100B THROUGH HOLE
      100a, 100b OPENING PORTION
      101 BENT PORTION
   110 BASE END PORTION
   120, 120A TIP PORTION
      121 INCLINED PORTION
      122 CURVED PORTION
   130 TAPERED SURFACE
   140 STEP PORTION
   150 UNEVENNESS
11 STOMACH WALL
12 BILE DUCT WALL
14 PUNCTURE HOLE
15 ABDOMINAL CAVITY

## Claims

1. An endoscopic puncture device (6) comprising:
a sheath (7) suitable to be inserted into an endoscope;
a hollow puncture needle (8) disposed in the sheath (7) and provided with a blade surface (82) at a tip thereof; and
a hollow resin molded body (10) provided in the puncture needle (8) on a distal side of the puncture needle (8) and at least partially disposed inside a tip opening portion (80a) of the puncture needle (8),
wherein the resin molded body (10) has a through hole (100) through the resin molded body (10) to insert a guide wire (4) and an opening portion (100a) formed at the distal end of the through hole (100), and
**characterized in that**
an edge portion of the opening portion (100a) is disposed along an edge portion of the tip opening portion (80a) and continuously extends along an entire circumference of the edge portion of the tip opening portion (80a).

2. The endoscopic puncture device (6) according to claim 1, wherein
the resin molded body (10) has a predetermined wall thickness such that the guide wire (4), when inserted therethrough, is centered with respect to a central axis of the puncture needle (8).

3. The endoscopic puncture device (6) according to claim 1 or 2, wherein the resin molded body (10) has an inclined portion (121) inclined in accordance with inclination of the blade surface (82).

4. The endoscopic puncture device (6) according to any one of claims 1 to 3, wherein an inner diameter of the resin molded body (10B) decreases toward a tip side of the resin molded body (10B).

5. The endoscopic puncture device (6) according to any one of claims 1 to 4, wherein a tapered surface (130) or a step portion (140) is formed on an outer peripheral surface of the resin molded body (10C, 10D).

## Patentansprüche

1. Endoskopische Punktionsvorrichtung (6), umfassend:
eine Hülse (7), die geeignet ist, in ein Endoskop eingeführt zu werden;
eine hohle Punktionsnadel (8), die in der Hülle (7) angeordnet und an ihrer Spitze mit einer Klingenfläche (82) versehen ist; und
einen hohlen Harzformkörper (10), der in der Punktionsnadel (8) an einer distalen Seite der Punktionsnadel (8) vorgesehen und zumindest teilweise innerhalb eines Spitzenöffnungsabschnitts (80a) der Punktionsnadel (8) angeordnet ist,
wobei
der Harzformkörper (10) ein Durchgangsloch (100) durch den Harzformkörper (10) zum Einführen eines Führungsdrahtes (4) und einen am distalen Ende des Durchgangslochs (100) ausgebildeten Öffnungsabschnitt (100a) aufweist, und
**dadurch gekennzeichnet, dass**
ein Kantenabschnitt des Öffnungsabschnitts (100a) entlang eines Kantenabschnitts des Spitzenöffnungsabschnitts (80a) angeordnet ist und sich kontinuierlich entlang eines gesamten Umfangs des Kantenabschnitts des Spitzenöffnungsabschnitts (80a) erstreckt.

2. Endoskopische Punktionsvorrichtung (6) nach Anspruch 1, wobei
der Harzformkörper (10) eine vorbestimmte Wandstärke aufweist, so dass der Führungsdraht (4), wenn er durch diesen hindurch eingeführt ist, in Bezug auf eine zentrale Achse der Punktionsnadel (8) zentriert ist.

3. Endoskopische Punktionsvorrichtung (6) nach Anspruch 1 oder 2, wobei der Harzformkörper (10) einen geneigten Abschnitt (121) aufweist, der entsprechend der Neigung der Klingenfläche (82) geneigt ist.

4. Endoskopische Punktionsvorrichtung (6) nach einem der Ansprüche 1 bis 3, wobei der Innendurchmesser des Harzformkörpers (10B) in Richtung einer Spitzenseite des Harzformkörpers (10B) abnimmt.

5. Endoskopische Punktionsvorrichtung (6) nach einem der Ansprüche 1 bis 4, wobei eine sich verjüngende Oberfläche (130) oder ein Stufenabschnitt (140) an einer äußeren Umfangsfläche des Harzformkörpers (10C, 10D) ausgebildet ist.

## Revendications

1. Dispositif de perforation endoscopique (6) comprenant :
une gaine (7) appropriée pour être insérée dans un endoscope ;
une aiguille de perforation creuse (8) disposée dans la gaine (7) et prévue avec une surface de lame (82) au niveau de sa pointe ; et
un corps creux moulé en résine (10) prévu dans l'aiguille de perforation (8) sur un côté distal de l'aiguille de perforation (8) et au moins partiellement disposé à l'intérieur d'une partie d'ouverture de pointe (80a) de l'aiguille de perforation (8),
dans lequel :
le corps moulé en résine (10) a un trou débouchant (100) à travers le corps moulé en résine (10) pour insérer un fil-guide (4) et une partie d'ouverture (100a) formée au niveau de l'extrémité distale du trou débouchant (100), et
**caractérisé en ce que** :
une partie de bord de la partie d'ouverture (100a) est disposée le long d'une partie de bord de la partie d'ouverture de pointe (80a) et s'étend, de manière continue, le long de toute la circonférence de la partie de bord de la partie d'ouverture de pointe (80a).

2. Dispositif de perforation endoscopique (6) selon la revendication 1, dans lequel :
le corps moulé en résine (10) a une épaisseur de paroi prédéterminée, de sorte que le fil-guide (4), lorsqu'il est inséré dans ce dernier, est centré par rapport à un axe central de l'aiguille de perforation (8).

3. Dispositif de perforation endoscopique (6) selon la revendication 1 ou 2, dans lequel le corps moulé en résine (10) a une partie inclinée (121) inclinée selon l'inclinaison de la surface de lame (82).

4. Dispositif de perforation endoscopique (6) selon l'une quelconque des revendications 1 à 3, dans lequel un diamètre interne du corps moulé en résine (10B) diminue vers un côté de pointe du corps moulé en résine (10B).

5. Dispositif de perforation endoscopique (6) selon l'une quelconque des revendications 1 à 4, dans lequel une surface progressivement rétrécie (130) ou une partie de gradin (140) est formée sur une surface périphérique externe du corps moulé en résine (10C, 10D).
